# EUROPEAN PATENT APPLICATION

(11) **EP 4 494 629 A1**
(43) Date of publication of application: **22.01.2025**
(21) Application number: 23186837.3
(22) Date of filing: 20.07.2023
(51) Int. Cl.: A61K 8/27

(54) **MEASUREMENT OF THE OLFACTORY EFFECT OF AN ORAL CARE PRODUCT**

(71) Applicant: Unilever IP Holdings B.V., 3013 AL Rotterdam (NL)
(72) Inventor: The designation of the inventor has not yet been filed
(74) Representative: Tansley, Sally Elizabeth

(57) **Abstract**

A method of measuring the olfactory effect of a zinc salt/oxide comprising the following steps:
a) creating an aqueous solution from an oral care composition.
b) creating a solution of a thiol generating compound.
c) adding the aqueous oral care composition solution a) to differing volumes of the thiol generating solution b) to form a mixture.
d) using the mixture c) to create an olfactory index profile.

## Description

### Field of the Invention

The invention relates to a method for measuring the effectiveness of zinc compounds in an oral care composition for mitigating thiol production in the mouth.

### Background of the Invention

The present invention concerns measuring the effectiveness of zinc compounds an oral care compositions in controlling odours in the mouth.

Oral care composition that freshen the breath have been on the commercial market for some time. Such compositions are usually formulated with perfumes and flavours that mask any odour. Oral care compositions also may comprise surfactants that help clean the mouth.

Certain ingredients such as zinc salts can complex volatile odours in the mouth. The present invention relates to a method for measuring the effectiveness of odours adsorbing compositions comprising zinc compounds.

### Summary of the Invention

The present application relates to a method of measuring the olfactory effect of a zinc salt/oxide comprising the following steps:
a) creating an aqueous solution from an oral care composition
b) creating a solution of a thiol generating compound
c) adding the aqueous oral care composition solution a) to differing volumes of thiol generating solution b) to form a mixture.
d) using the mixture c) to create an olfactory index profile.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any ranges of values, any upper value can be associated with any particular lower value.

Where a feature is disclosed with respect to a particular aspect of the invention (for example a composition of the invention), such disclosure is also to be considered to apply to any other aspect of the invention (for example a method of the invention) *mutatis mutandis.*

Oral care composition are any treatments used to treat or enhance the oral cavity and teeth, preferably they are used to clean the surfaces of the oral cavity in particular the teeth.

Accordingly, preferred product forms for compositions for use by the invention are those which are suitable for brushing and/or rinsing the surfaces of the oral cavity.

The composition for use by the invention is most preferably in the form of a dentifrice. The term "dentifrice" denotes an oral composition which is used to clean the surfaces of the oral cavity. Such a composition is not intentionally swallowed for purposes of systemic administration of therapeutic agents, but is applied to the oral cavity, used to treat the oral cavity and then expectorated. Typically such a composition is used in conjunction with a cleaning implement such as a toothbrush, usually by applying it to the bristles of the toothbrush and then brushing the accessible surfaces of the oral cavity.

Preferably the thiol generating compound is hydrogen sulphide.

Preferably the thiol generating solution, particularly the hydrogen sulphide solution comprises from 2 to 30 µg/ml, more preferably rom 5 to 15 µg/ml of thiol producing compound.

The olfactory profile index may be created by smelling the head space above the mixture d) to assess whether there is any odour or by measuring the presence of thiol in the head space using an Oral Chroma instrument.

It is beneficial if the thiol level is measured in 1ml of headspace above mixture d)

A method in which the aqueous solution of oral care composition a) is prepared by addition of toothpaste iso water to make a slurry, the slurry is then homogenised and centrifuged followed by removal of residual solids.

The slurry is preferably water-based, preferably comprising at least 50 wt% of the total slurry of water, more preferably at least 75wt%.

A method according to any preceding claim in which the oral care composition is a toothpaste composition.

Accordingly, preferred product forms for compositions of the invention are those which are suitable for brushing and/or rinsing the surfaces of the oral cavity.

The composition of the invention is most preferably in the form of a dentifrice. The term "dentifrice" denotes an oral composition which is used to clean the surfaces of the oral cavity. Such a composition is not intentionally swallowed for purposes of systemic administration of therapeutic agents, but is applied to the oral cavity, used to treat the oral cavity and then expectorated. Typically such a composition is used in conjunction with a cleaning implement such as a toothbrush, usually by applying it to the bristles of the toothbrush and then brushing the accessible surfaces of the oral cavity.

Preferably the dentifrice/toothpaste is in the form of an extrudable semi-solid such as a cream, paste or gel (or mixture thereof).

It is preferable if the zinc compound is zinc citrate or more preferably zinc sulphate.

A composition according to the invention (such as a dentifrice/toothpaste) will generally contain further ingredients to enhance performance and/or consumer acceptability, in addition to the ingredients specified above.

Compositions according to the invention, particularly toothpastes, preferably comprise particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition.

In one embodiment of the invention the composition comprises a silica based abrasive. The preferred abrasive silicas used in the present invention is a silica with a low refractive index. It may be used as the sole abrasive silica, or in conjunction with a low level of other abrasive silicas, e.g. those according to EP 236070. The low refractive index silicas, used as abrasives in the present invention are preferably silicas with an apparent refractive index (R.I.) in the range of 1.41 -1.47, preferably 1.435 - 1.445, preferably having a weight mean particle size of between 5 and 15 mm, a BET (nitrogen) surface area of between 10 and 100 m²/g and an oil absorption of about 70 - 150 cm³/100 g, but abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Evonik, Sorbosil AC 77 ex PQ Corporation (having an R.I. of approximately 1.440). The amount of these silicas in the composition generally ranges from 5-60% by weight, usually 5-20% by weight.

An alternative suitable abrasive is calcium carbonate. Natural calcium carbonate abrasive is typically a finely ground limestone which may optionally be refined or partially refined to remove impurities. The material preferably has an average particle size of less than 10 microns, e.g., 3-7 microns, e.g., about 5.5 microns. For example, a small particle silica may have an average particle size (D50) of 2.5 -4.5 microns.. There are different polymorphs of natural calcium carbonate, e.g., calcite, aragonite and vaterite, calcite being preferred for purposes of this invention.

Precipitated calcium carbonate has a different crystal structure from natural calcium carbonate. It is generally more friable and more porous, thus having lower abrasivity and higher water absorption. For use in the present invention, the particles are small, e.g., having an average particle size of 1-5 microns, and e.g., no more than 0.1 percent, preferably no more than 0.05 percent by weight of particles which would not pass through a 325 mesh. The particles may for example have a D50 of 3-6 microns, for example 3.8-4.9, e.g., about 4.3; a D50 of 1-4 microns, e.g., 2.2-2.6 microns, e.g., about 2.4 microns, and a D10 of 1-2 microns, e.g., 1.2-1.4, e.g., about 1.3 microns. The particles have relatively high water absorption, e.g., at least 25 g/100 g, e.g., 30-70 g/100 g.

In some embodiments, additional calcium-containing abrasives, for example calcium phosphate abrasive, e.g., tricalcium phosphate, hydroxyapatite or dicalcium phosphate dihydrate or calcium pyrophosphate, and/or silica abrasives, sodium metaphosphate, potassium metaphosphate, aluminum silicate, calcined alumina, or other siliceous materials, or combinations thereof are used. The composition, particularly if a toothpaste preferably comprises an inorganic or a natural or synthetic thickener or gelling agent in proportions of about 0.10 to about 15% by weight depending on the material chosen. These proportions of thickeners in the dentifrice compositions of the present invention form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Corporation under the trade designation Zeodent 165, Irish moss, iota-carrageenan, gum tragacanth, and polyvinylpyrrolidone.

The invention will now be illustrated by the following non-limiting Examples

### Examples

### Formulation:

Two toothpaste formulations were prepared as outlined in the table below:

| | **Example 1/wt%** | **Example 2/ wt%** |
|---|---|---|
| Sorbitol (70%) | 58 | 58 |
| Thickening Silica | 8.5 | 8.5 |
| Abrasive Silica | 10.6 | 10.6 |
| SLS | 2.7 | 2.7 |
| Zinc Citrate trihydrate | 1 | 2 |
| Cellulose Gum | 0.7 | 0.7 |
| Other Minors | 1.9 | 1.9 |
| Water | To 100% | To 100% |

### Assessment:

A 1:1 slurry of toothpaste and water was prepared by combining 20g of toothpaste and 20g deionised water using a Fastprep-24 mixer supplied by MP biomedical. The mixture was homogenised. The mixture was then spun in a centrifuge at 6000rpm for 15 minutes to remove the solids. The separated liquid was transferred to another container.

A hydrogen sulphide solution was prepared using Aroxa 72ug hydrogen sulphide flavour tablets produced by Cara Technologies. The tablet contents were placed in a vial and made up to 7.2ml in mass with deionised water to make a 10ug/ml hydrogen sulphide solution.

To a second vial 0.01g of toothpaste slurry liquid mixture was added. To this vial 0.5ml or 1ml aliquots of hydrogen sulphide solution were added and the vial resealed. The contents were shaken by hand to mix and left for two minutes to equilibrate. After two minutes the lid was removed, and the head spaced smelled. The presence of a thiol in the headspace of the vial can be detected as a boiled or rotten egg like aroma.

Comparative Example: Colgate Max Fresh Sourced from India market.

| | | |
|---|---|---|
| H2S Solution Volume | 0.5ml | 1ml |
| Sulphide smell present | Yes | Yes |

### Example 1:

| | | | | | | |
|---|---|---|---|---|---|---|
| H2S Solution Volume | 0.5ml | 1ml | 1.5ml | 2ml | 2.5ml | 3ml |
| Sulphide smell present | No | No | No | No | Yes | Yes |

### Example 2:

| | | | | | | | | | |
|---|---|---|---|---|---|---|---|---|---|
| H2S Solution Volume | 1ml | 2ml | 3ml | 4ml | 5ml | 6ml | 7ml | 8ml | 8.5ml |
| Sulphide smell present | No | No | No | No | No | No | No | Yes | Yes |

The data shows that an olfactory approach can successfully be used to measure the ability of zinc salts in toothpaste to bind hydrogen sulphide.

### Instrument assessment

### Assessment:

A 1:1 slurry of toothpaste and water was prepared by combining 20g of toothpaste and 20g deionised water using a Fastprep-24 mixer supplied by MP biomedical. The mixture was homogenised at 6m2 for 20 seconds. The mixture was then spun in a centrifuge at 6000rpm for 15 minutes to remove the solids. The separated liquid was transferred to another container.

A hydrogen sulphide solution was prepared using Aroxa 72ug hydrogen sulphide flavour tablets produced by Cara Technologies. The tablet contents were placed in a vial and made up to 7.2ml in mass with deionised water to make a 10ug/ml hydrogen sulphide solution.

To a second vial 0.01g of toothpaste slurry liquid mixture was added. To this vial 0.5ml or 1ml aliquots of hydrogen sulphide solution were added and the vial resealed. The contents were shaken by hand to mix and left for two minutes to equilibrate. After two minutes 1ml of the headspace was removed with a syringe and an Oral Chroma instrument from Nissha Co Ltd (Japan) was used to measure hydrogen sulphide in the headspace.

Comparative Example: Colgate Max Fresh Sourced for India market.

| | | |
|---|---|---|
| H2S Solution Volume | 0.5ml | 1ml |
| Sulphide response/ppb | 3483 | 4474 |

### Example 1:

| | | | | | | |
|---|---|---|---|---|---|---|
| H2S Solution Volume | 0.5ml | 1ml | 1.5ml | 2ml | 2.5ml | 3ml |
| Sulphide response/ppb | 9 | 8 | 15 | 105 | 344 | 609 |

### Example 2:

| | | | | | | | | | |
|---|---|---|---|---|---|---|---|---|---|
| H2S Solution Volume | 1ml | 2ml | 3ml | 4ml | 5ml | 6ml | 7ml | 8ml | 8.5ml |
| Sulphide response/ppb | 17 | 21 | 21 | 25 | 27 | 29 | 63 | 237 | 601 |

The data shows that an Oral Chroma approach can successfully be used to measure the ability of zinc salts in toothpaste to bind hydrogen sulphide.

### Zinc assessment

### Formulation:

A toothpaste formulation was prepared as outlined in the table below:

### Example 3

| | **Example 1/wt%** |
|---|---|
| Sorbitol (70%) | 58 |
| Thickening Silica | 8.5 |
| Abrasive Silica | 10.6 |
| SLS | 2.7 |
| Zinc Sulphate heptahydrate | 0.8 |
| Glycine | 0.3 |
| Cellulose Gum | 0.7 |
| Other Minors | 1.9 |
| Water | To 100% |

### Assessment:

A 1:1 slurry of toothpaste and water was prepared by combining 20g of toothpaste and 20g deionised water using a Fastprep-24 mixer supplied by MP biomedical. The mixture was homogenised. The mixture was then spun in a centrifuge at 6000rpm for 15 minutes to remove the solids. The separated liquid was transferred to another container.

A hydrogen sulphide solution was prepared using Aroxa 72ug hydrogen sulphide flavour tablets produced by Cara Technologies. The tablet contents were placed in a vial and made up to 7.2ml in mass with deionised water to make a 10ug/ml hydrogen sulphide solution.

To a second vial 0.01g of toothpaste slurry liquid mixture was added. To this vial 0.5ml or 1ml aliquots of hydrogen sulphide solution were added and the vial resealed. The contents were shaken by hand to mix and left for two minutes to equilibrate. After two minutes 1ml of the headspace was removed with a syringe and an Oral Chroma instrument from Nissha Co Ltd (Japan) was used to measure hydrogen sulphide in the headspace.

Comparative Example: Colgate Max Fresh Sourced for India market.

| | | |
|---|---|---|
| H2S Solution Volume | 0.5ml | 1ml |
| Sulphide response/ppb | 3483 | 4474 |

### Example 3:

| | | | | | | |
|---|---|---|---|---|---|---|
| H2S Solution Volume | 1ml | 2ml | 3ml | 4ml | 5ml | 6ml |
| Sulphide response/ppb | 15 | 29 | 23 | 31 | 128 | 1630 |

A second assessment was carried out using an olfactory approach. To a vial 0.01g of toothpaste slurry liquid mixture was added. To this vial 0.5ml or 1ml aliquots of hydrogen sulphide solution were added and the vial resealed. The contents were shaken by hand to mix and left for two minutes to equilibrate. After two minutes the lid was removed, and the head spaced smelled. The presence of a thiol in the headspace of the vial can be detected as a boiled or rotten egg like aroma. Comparative Example: Colgate Max Fresh Sourced for India market.

| | | |
|---|---|---|
| H2S Solution Volume | 0.5ml | 1ml |
| Sulphide smell present | Yes | Yes |

### Example 3:

| | | | | | | |
|---|---|---|---|---|---|---|
| H2S Solution Volume | 1ml | 2ml | 3ml | 4ml | 5ml | 6ml |
| Sulphide smell present | No | No | No | No | No | Yes |

The data shows that an Oral Chroma or olfactory approach can successfully be used to measure the ability of zinc salts in toothpaste to bind hydrogen sulphide.

## Claims

1. A method of measuring the olfactory effect of a zinc salt/oxide comprising the following steps:
a) creating an aqueous solution from an oral care composition.
b) creating a solution of a thiol generating compound.
c) adding the aqueous oral care composition solution a) to differing volumes of the thiol generating solution b) to form a mixture.
d) using the mixture c) to create an olfactory index profile.

2. A method according to claim 1 in which the thiol generating compound is hydrogen sulphide.

3. A method according to claim 2 in which the hydrogen sulphide solution comprises from 5 to 15 µg/ml of thiol producing compound.

4. A method according to any according preceding claim in which the zinc compound is zinc citrate, more preferably zinc sulphate.

5. A method according to any preceding claim in which the olfactory profile index is created by measuring the presence of thiol in the head space above the mixture d).

6. A method according to any preceding claim in which the level of thiol is measured using an Oral Chroma instrument.

7. A method according to any preceding claim in which the thiol level is measured in 1ml of headspace above mixture d)

8. A method according to any preceding claim in which the oral care composition is a toothpaste composition.

9. A method according to claim 8 in which the aqueous solution of oral care composition a) is prepared by addition of toothpaste iso water to make a slurry, the slurry is then homogenised and centrifuged followed by removal of residual solids.
